# FASCICULE DE BREVET EUROPEEN

(11) **EP 0 865 254 B1**
(45) Date de publication et mention de la délivrance du brevet: **16.01.2002**
(21) Numéro de dépôt: 96938276.1
(22) Date de dépôt: 08.11.1996
(51) Int. Cl.: A61B 10/00

(54) **SAC JETABLE POUR LE RECUEIL DE MATIERES FECALES**
WEGWERFBEUTEL ZUR AUFNAHME VON FÄKALIEN
DISPOSABLE BAG FOR COLLECTING FAECAL MATTER

(30) Priorité: 08.11.1995 FR 9513191
(43) Date de publication de la demande: 23.09.1998
(73) Titulaire: Le Fourn, Gilbert, 29800 Plouedern (FR)
(72) Inventeur: Le Fourn, Gilbert, 29800 Plouedern (FR)
(74) Mandataire: Thibon-Littaye, Annick
(86) Numéro de dépôt international: FR9601759
(87) Numéro de publication internationale: WO9717019

(56) Documents cités:
- US-A- 3 346 883
- US-A- 3 588 921
- US-A- 4 007 741
- US-A- 4 445 235
- US-A- 5 337 426

## Description

La présente invention concerne un sac jetable qui est conçu pour s'adapter sur des cuvettes sanitaires et qui est destiné, en particulier à des fins d'analyse médicale.

Actuellement, lorsqu'un médecin prescrit une analyse de selles, on fournit au patient une spatule destinée à prélever des échantillons de selles et à la déposer dans une boîte, généralement en matière synthétique, qui peut être envoyée à un laboratoire d'analyse.

Il n'existe aucun moyen pratique pour réaliser facilement et sans désagrément cette opération de prélèvement d'échantillons de selles, si bien que ces analyses sont mal acceptées par les patients, alors que leur intérêt est souvent très important, car elles constituent une opération de dépistage de maladies graves.

Il est donc souhaitable de disposer d'un moyen pratique pour le prélèvement de selles, afin que les analyses de selles soient bien acceptées par les patients et deviennent un examen banal. Par ailleurs, dans le cas d'examens bactériologiques en particulier, il est nécessaire que les selles puissent être prélevées de manière stérile sans être souillées par des germes étrangers.

Les dispositifs qui ont pu être proposés à ce jour en ce sens n'ont pas donné satisfaction, et de ce fait ils n'ont pas été mis en oeuvre en pratique. On pense ici en particulier à des feuilles en papier biodégradable, dans lesquelles le papier est en une matière suffisamment extensible pour pouvoir se dilater en s'incurvant sous le poids des matières reçues, de manière à former une sorte de poche.

A partir de ce principe, il est apparu impossible, à l'échelle industrielle nécessaire, de mettre dans le commerce un dispositif de collecte des matières fécales qui convienne en toutes circonstances, que ce soit d'un individu à un autre ou d'un jour à l'autre chez le même patient. La plupart du temps, mais aussi de manière imprévisible, la matière de la feuille se déchire sous le poids des matières fécales, et ce d'autant plus qu'elle devient humide.

Le document US-A-4445235 (D1) décrit un sac jetable 46 pour le recueil de matières fécales, réalisé en une feuille de matière biodégradable non extensible pouvant être évacuée par les WC et perméable aux liquides (voir col.4, lignes 13-20), qui présente, sur deux rabats symétriques, au moins un élément adhésif 53 en sous-face destiné à une fixation provisoire sur une cuvette de WC.

Par ailleurs, le document US-A-3588921 suggère de réaliser un sac jetable pour le recueil de matières fécales sous forme d'un paquet plat pliable.

Pour résoudre ces difficultés et répondre aux besoins de la pratique, la présente invention propose un sac jetable selon la revendication 1 pour le recueil de matières fécales, en particulier en vue d'analyses médicales, qui est réalisé en une feuille de matière biodégradable non extensible pouvant être évacuée par les W-C et perméable aux liquides, cette feuille constituant un réservoir dépliable qui est plat à l'état plié et qui présente, en sous-face de deux rabats symétriques, au moins un élément adhésif destiné à une fixation provisoire sur une cuvette de W-C.

Par matière non extensible, on désigne ici, comme il s'entend de soi, une matière qui, dans les conditions d'usage ne se déforme pas par étirement et dilatation sous le poids des matières, si bien que le sac ou réservoir est simplement formé par dépliement du sac selon l'invention, et non pas par extension de la feuille elle-même, sous l'effet d'un effort manuel ou efforts liés au poids des matières reçues, alors même que la feuille de matière biodégradable se trouve humidifiée par un liquide aqueux.

Grâce à l'invention, l'opération de prélèvement peut être facilement réalisée lorsque le patient se rend aux toilettes. Il lui suffit de placer et fixer le sac jetable sur la cuvette de W-C, et les matières fécales expulsées sont recueillies dans le sac jetable au lieu de tomber au fond de la cuvette de W-C. Le patient peut alors facilement prélever un ou plusieurs échantillons de matières fécales, sans risque d'endommager la feuille et de voir le sac se déchirer. Il peut ensuite décoller l'élément adhésif du rebord de la cuvette et tirer la chasse d'eau pour évacuer le sac jetable et le reste de son contenu.

Selon une autre caractéristique de l'invention, la matière biodégradable est du type des tissus ou papiers, comportant des couches multiples de fibres non tissées entrelacées pour former un feutre, comme c'est le cas de la ouate de cellulose, encore qu'il existe dans le commerce d'autres fibres que les fibres cellulosiques qui sont de nature organique synthétique et biodégradables et qui sont propres à remplir les mêmes fonctions dans la constitution d'un feutre de fibres non tissées biodégradable.

Une telle matière, telle la ouate de cellulose, qui est connue et utilisée, en particulier, pour réaliser des articles d'hygiène, forme des feuilles qui présentent une résistance mécanique suffisante pour ne pas se déchirer sous le poids des matières fécales, même à l'état humide. A titre d'exemple convenant spécialement ici, on peut utiliser une ouate du type des matières servant à réaliser des essuie-mains de bonne qualité, c'est-à-dire une matière qui peut absorber dans son épaisseur, sans partir en lambeaux, toute l'eau présente sur les deux mains d'une personne qui vient de se laver les mains.

Selon l'invention, le sac jetable est réalisé sous la forme d'une bande pliée sur elle-même en deux moitiés selon une ligne de pliage de fond transversale, perpendiculaire à la longueur de ladite bande, les bords longitudinaux des deux moitiés de ladite bande étant réunis, de chaque côté, par un élément triangulaire, au moins sur leur partie adjacente à la ligne de pliage de fond, chacun des éléments triangulaires présentant une ligne de pliage latérale disposée selon la bissectrice de l'angle adjacent à la ligne de pliage de fond. Cette forme de réalisation est facile à fabriquer, et elle permet d'obtenir un sac qui se met en forme automatiquement quand il se déplie à l'usage.

Avantageusement, le pliage selon la ligne de pliage latérale est réalisé vers l'extérieur. De cette manière, le sac se déploie automatiquement dès que l'utilisateur tire sur les deux moitiés de bande pour les écarter, l'utilisateur n'ayant pas à mettre la main dans le réservoir.

Selon encore une autre caractéristique de l'invention, le sac jetable comporte deux lignes de pliage longitudinales correspondant aux côtés non libres de l'élément triangulaire de manière à pouvoir rabattre les éléments triangulaires pliés en deux sur la bande, lorsque le sac est à l'état plié.

Avantageusement, chaque moitié de la bande comporte une ligne de pliage transversale de rabat aménagée au-dessus de sa partie reliée à l'élément triangulaire de manière à déterminer un rabat sur la face inférieure duquel est disposé l'élément adhésif.

Ceci permet de fixer le sac sur les deux côtés d'une cuvette de W-C du type à l'anglaise, en particulier sur la lunette d'abattant si la cuvette en comporte une.

Avantageusement, l'élément adhésif est une bande adhésive double-face collée sur la face inférieure de la bordure ou du rabat et la face supérieure du rebord ou rabat présente un indicateur coloré.

Grâce à cette disposition, l'utilisateur est guidé pour déployer le sac dans le bon sens.

Le sac jetable suivant l'invention peut facilement être fabriqué à partir d'une bande continue de feuille non extensible biodégradable dans laquelle on découpe un contour symétrique par rapport à une ligne transversale de la feuille correspondant à la ligne de pliage de fond, ledit contour étant plié selon ladite ligne transversale et les bords latéraux dudit contour étant fixés l'un sur l'autre.

D'autres caractéristiques et avantages de l'invention ressortiront de la description qui suit d'un mode de réalisation, faite à titre non limitatif en se référant aux dessins ci-annexés, sur lesquels :
- la figure 1 est une vue en perspective d'une cuvette de W-C équipée d'un sac jetable selon l'invention,
- la figure 2 est une vue de dessus de la cuvette de W-C de la figure 1,
- la figure 3 est une vue en coupe transversale de la cuvette de W-C de la figure 1,
- la figure 4 représente, en vue de profil le sac jetable à l'état déplié,
- la figure 5 représente le sac jetable plié en selon la ligne de pliage de fond, et
- la figure 6 illustre la fabrication du sac jetable.

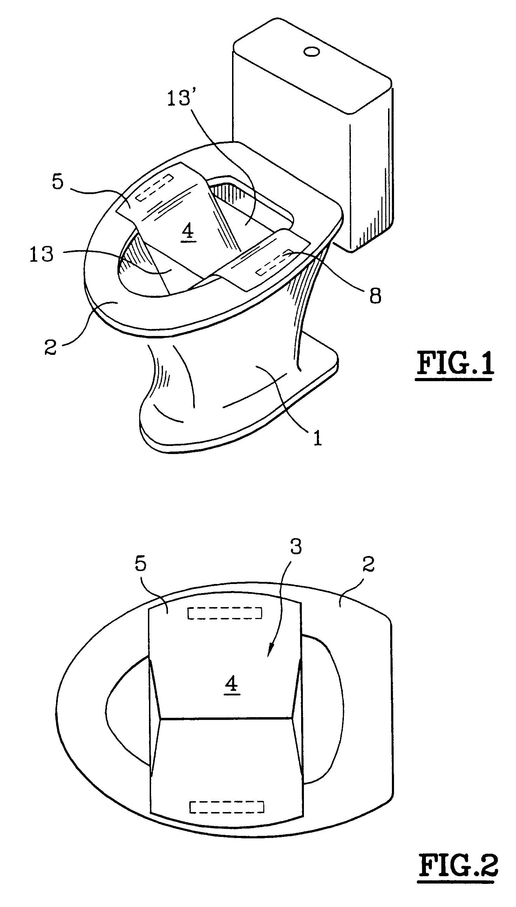

On voit sur les figures 1 à 3 une cuvette de W-C 1 du type à l'anglaise munie d'un abattant 2 dans sa partie formant lunette, et non couvercle. Sur cet abattant 2 est fixé un sac jetable 3, qui est essentiellement constitué d'un réservoir dépliable 4, muni de deux rabats 5 qui sont appliqués sur l'abattant 2 et y sont maintenus par adhérence par contact.

A cet effet, chaque rabat 5 comporte en sous-face un élément adhésif 6, disposé sur sa face inférieure 7 venant en contact avec l'abattant 2. Avantageusement, on prévoit un indicateur coloré 8 sur la face supérieure du rabat 5 du sac jetable 3. Cet indicateur 8 permet de repérer la face adhésive du rabat 5 lorsque le sac jetable est à l'état plié, afin que l'utilisateur présente le sac jetable dans le bon sens pour le mettre en place.

Le sac jetable selon l'invention est réalisé en une matière biodégradable pouvant être évacuée par les W-C pour destruction et néanmoins perméable aux liquides. Cette matière est telle qu'elle présente un bon compromis entre, d'une part, la capacité d'absorption d'eau nécessaire pour la biodégradabilité et une évacuation aisée, et d'autre part, la préservation d'une résistance mécanique suffisante à l'état humide pour ne pas se déchirer sous la pression statique et dynamique de la quantité maximale de selles que peut produire un individu. C'est pourquoi elle est choisie essentiellement non extensible dans les conditions d'emploi pratique.

On peut par exemple utiliser une matière qui comporte des couches multiples sous forme d'un feutre de fibres non tissées, telle que la ouate de cellulose utilisée pour fabriquer des articles absorbants de faible épaisseur, comme les serviettes à usage hygiénique vendues notamment pour servir d'essuie-mains.

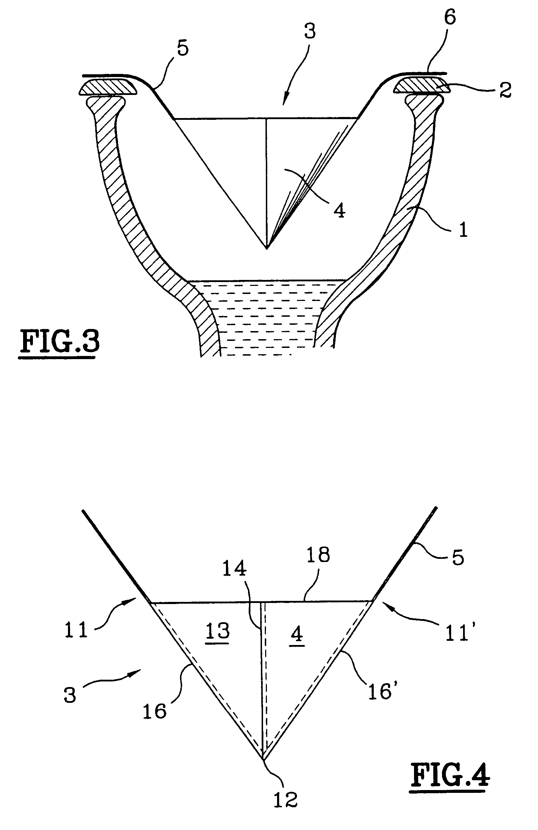

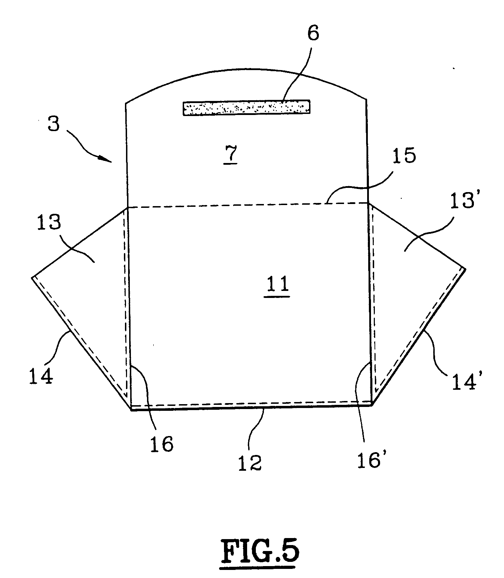

Sur les figures 4 et 5, on a représenté en détail un mode de réalisation du sac jetable selon la présente invention. Il se présente sous la forme d'une feuille en bande continue qui est repliée sur elle-même en deux moitiés 11 et 11' selon une ligne de pliage transversale de fond 12. Les deux moitiés 11 et 11' sont reliées sur une partie de leur longueur, de chaque côté, par un élément triangulaire 13, respectivement 13', adjacent à la ligne de pliage de fond 12.

Chaque élément triangulaire 13, 13' comporte une ligne de pliage latérale 14, respectivement 14', qui correspond à la bissectrice de l'angle adjacent à la ligne de pliage de fond 12. Le pliage du sac selon cette ligne 13, 13' est réalisé vers l'extérieur, c'est-à-dire comme représenté sur la figure 5, de telle sorte qu'à l'état plié, l'élément triangulaire 13, 13' se trouve à l'extérieur de la bande 11, 11'.

Chaque bande 11, 11' comporte une ligne de pliage transversale de rabat 15, respectivement 15', qui permet d'obtenir le rabat 5. Pour limiter l'encombrement du sac à l'état plié, on prévoit, de chaque côté de la bande 11, 11', deux lignes de pliage longitudinales 16, respectivement 16', qui permettent, à partir de l'état de pliage de la figure 5, de rabattre les éléments triangulaires 13, 13', déjà repliés sur eux-mêmes, sur la bande 11, 11', de manière que la largeur du sac à l'état plié soit équivalent à la largeur de la bande.

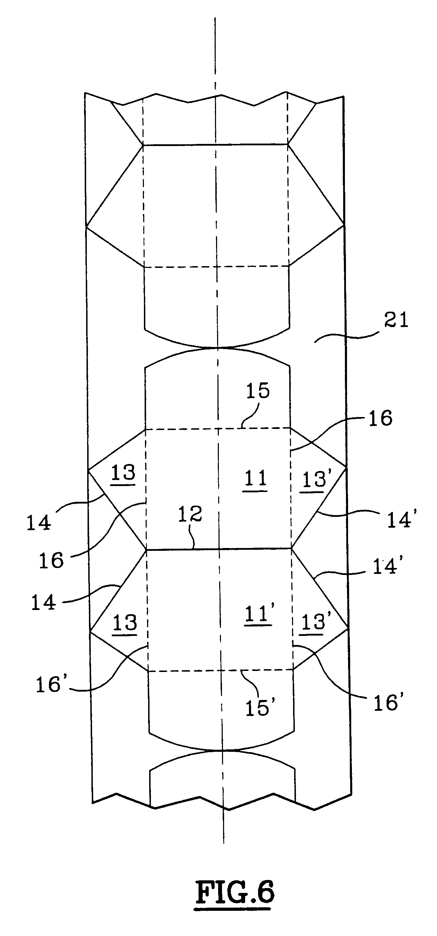

La figure 6 illustre un procédé de fabrication du sac jetable qui vient d'être décrit. Il est réalisé à partir d'une feuille 21, en forme de bande ou ruban entre deux bords parallèles dans laquelle on découpe un patron suivant un contour symétrique par rapport à l'axe longitudinal de la feuille 21 et à la ligne transversale de pliage de fond 12. De chaque côté de la feuille 21, on découpe une moitié des éléments triangulaires 13 et 13' divisés selon la ligne de pliage latérale 14, 14'.

Après l'opération de découpe, on réalise les lignes de pliage 15, 15', 16 et 16'. Ensuite, les deux moitiés de bande 11 et 11' sont repliées l'une sur l'autre par pliage selon la ligne 12. On assemble alors les moitiés des éléments triangulaires 13 et 13' selon la ligne de pliage latérale 14, 14'. Pour réaliser cet assemblage, par exemple par collage, on peut prévoir de laisser sur les lignes de pliage latérales 16 et 16' une petite bande de matière de la feuille afin d'obtenir un recouvrement.

On observera que sur les zones de collage, la résistance de la matière de base utilisée se trouve naturellement renforcée. Dans la même optique, on peut avantageusement prévoir de renforcer le sac localement selon la ligne de pliage 12, par une bande supplémentaire collée. Dans tous les cas, la solution préconisée est optimale en ce qu'elle répond à la fois aux objectifs de fiabilité d'emploi et à une fabrication à coût réduit, satisfaisant aux besoins de la faisabilité économique, tant en termes de matière consommable qu'en main d'oeuvre.

A titre d'exemple, le demandeur a réalisé un sac jetable présentant les dimensions suivantes. La largeur de la bande ou ruban de feuille 11 est de 19 cm, la longueur totale de chaque moitié 11, 11' est de 26,5 cm, la longueur du côté libre 18 des éléments triangulaires est de 19 cm, la longueur des autres côtés est de 15,5 cm, de telle sorte que la longueur des rabats est de 11 cm.

La matière utilisée est une ouate de cellulose en deux couches, présentant un poids spécifique de 2 x 2,45 g/m². Sa capacité d'absorption en eau est de 230 g/m². La résistance à la traction (en grammes par échantillon pris entre deux mâchoires espacées de 50 cm) est à l'état sec de 3380 g dans le sens machine et de 2660 g dans le sens transversal, à l'état sec, et de 630 dans le sens transversal à l'état humide. L'allongement après rupture lors du test précédent est de 24 %.

Le sac jetable selon l'invention est fourni à l'état plié dans un emballage protecteur, qui peut renfermer également une spatule de prélèvement, de manière à respecter les règles d'hygiène. Cet emballage est stérile quand il est destiné à des examens bactériologiques ; s'il s'agit de recherche de sang, d'examens chimiques non bactériologiques ou d'un simple usage de confort sanitaire, les sacs peuvent être livrés en vrac.

L'utilisateur sort le sac jetable de son emballage, applique la face inférieure adhésive de la bordure sur l'abattant. Eventuellement, il déplie le réservoir 4 manuellement pour lui donner la forme représentée à la figure 1. Il lui suffit d'écarter l'un de l'autre les deux rebords, comme il doit le faire pour les apposer sur la lunette et les bords de la cuvette, et le sac se déploie pour se creuser en formant réservoir. Compte tenu du dimensionnement préconisé, il ne risque pas pour autant de se mouiller les mains ou d'immerger la matière du sac dans l'eau qui remplit en général le fond de la cuvette. Le sac se déploie naturellement, sans qu'il y ait lieu d'exercer des efforts contrôlés de traction dans la limite de la résistance à la rupture de la feuille.

Les matières fécales sont ensuite recueillies dans le réservoir 4. Elles se trouvent à un niveau nettement plus élevé que le fond de la cuvette de W-C, donc hors d'eau, ce qui facilite le prélèvement d'un ou plusieurs échantillons de matières fécales au moyen de la spatule, les matières fécales étant disponibles à sec.

Les échantillons ainsi prélevés sont disposés dans l'emballage, avantageusement une boîte prête à être transmise à un centre d'examens, par exemple par la poste. Avant de tirer la chasse d'eau, l'utilisateur décolle la bordure du sac jetable et celui-ci tombe de lui-même au fond de la cuvette de W-C. Il peut donc alors être évacué normalement avec le reste de son contenu dans les circuits d'égoût.

On voit que l'invention permet de réaliser de manière simple et hygiénique le prélèvement de matières fécales à des fins d'analyse médicale. Ce prélèvement ne présente aucune gêne pour les patients, qui n'auront donc plus de réticence à se soumettre à des examens importants, en particulier des examens de dépistage par la présence de sang dans les selles.

Le sac jetable selon l'invention peut être fabriqué à faible prix et il pourra être distribué aux médecins généralistes, aux gastro-entérologues, aux pédiatres, aux hôpitaux, aux pharmacies, aux laboratoires d'analyses médicales, aux cliniques et aux centres de dépistage systématiques tels que ceux des caisses d'assurance maladie.

Le même sac jetable selon l'invention peut être utilisé en tirant bénéfice de ses caractéristiques sans qu'il y ait nécessairement prélèvement des matières fécales pour analyse. En particulier, il peut être commercialisé pour des applications qui ne sont pas toujours considérées du domaine médical à proprement parler, mais du domaine sanitaire plus généralement, par exemple pour apporter à l'utilisateur des toilettes, un confort supplémentaire en lui évitant les risques de recevoir des projections de produits versés dans la cuvette, tels que des produits désinfectants.

La description ci-dessus n'a été fournie qu'à titre illustratif et l'on peut y apporter des modifications ou variantes sans sortir du cadre de la présente invention. En particulier, on peut utiliser des matières différentes pour constituer la feuille en bande ou ruban dans lequel le patron du sac est découpé, afin d'adapter leur choix en fonction des conditions d'emploi prévues.

On peut notamment, tout en conservant un bon compromis entre la capacité d'absorption d'eau et la perméabilité à l'eau, ainsi que le caractère biodégradable, prévoir d'améliorer la résistance mécanique, notamment à l'état humide, en faisant appel à des matériaux multi-couches combinant des couches de fibres non tissées enchevêtrées en feutre avec des couches intercalaires de longues fibres en polyester parallèles entre elles et orientées dans des directions différentes d'une couche à l'autre.

## Revendications

1. Sac jetable pour le recueil de matières fécales, en particulier en vue d'analyses médicales, réalisé en une feuille de matière biodégradable pouvant être évacuée par les W-C, ladite matière étant choisie non extensible et perméable aux liquides, et qui présente, sur deux rabats symétriques (5), au moins un élément adhésif (6) en sous-face destiné à une fixation provisoire sur une cuvette de W-C (1), **caractérisé en ce qu'**il est configuré sous la forme d'un réservoir dépliable (4) qui est plat à l'état plié et **en ce qu'**il est réalisé sous la forme d'une bande de feuille pliée sur elle-même en deux moitiés (11, 11') selon une ligne de pliage de fond (12) transversale perpendiculaire à la longueur de ladite bande, les bords longitudinaux des deux moitiés de ladite bande étant réunis, de chaque côté, par un élément triangulaire (13, 13') au moins sur leur partie adjacente à la ligne de pliage de fond (12), chacun des éléments triangulaires (13, 13') présentant une ligne de pliage latérale (14, 14') disposée selon la bissectrice de l'angle adjacent à la ligne de pliage de fond (12).

2. Sac jetable selon la revendication 1, **caractérisé en ce que** la matière biodégradable comporte des couches multiples de fibres non tissées enchevêtrées formant un feutre.

3. Sac jetable selon la revendication 1, caractérisé en ce le pliage selon la ligne de pliage latérale (14, 14') est réalisé vers l'extérieur.

4. Sac jetable selon la revendication 3 ou 4, **caractérisé en ce qu'**il comporte deux lignes de pliage longitudinales (16, 16') correspondant aux côtés non libres de l'élément triangulaire (13, 13') de manière à pouvoir rabattre les éléments triangulaires pliés en deux sur la bande, lorsque le sac est à l'état plié.

5. Sac jetable selon la revendication 3, **caractérisé en ce que** chaque moitié (11, 11') de la bande comporte une ligne de pliage transversale (15, 15') de rabat ménagée au-dessus de sa partie reliée à l'élément triangulaire, de manière à déterminer un rabat (5) sur la face inférieure duquel est disposé l'élément adhésif (6).

6. Sac jetable selon l'une quelconque des revendications précédentes, **caractérisé en ce que** l'élément adhésif (6) est une bande adhésive double-face collée sur la face inférieure du rabat (5).

7. Sac jetable selon la revendications 5, **caractérisé en ce que** la face supérieure du rabat (5) présente un indicateur coloré (8).

8. Sac jetable selon l'une quelconque des revendications 3 à 8, **caractérisé en ce qu'**il est réalisé à partir d'une feuille en bande ou ruban continu (21) de matière dans lequel on découpe un contour symétrique par rapport à une ligne transversale du ruban correspondant à la ligne de pliage de fond (12), ledit contour étant plié selon ladite ligne transversale (12) et les bords latéraux dudit contour étant fixés l'un sur l'autre.

9. Sac jetable selon l'une quelconque des revendications 1 à 9, **caractérisé en ce que** la matière de ladite feuille est faite d'ouate de cellulose.

## Patentansprüche

1. Wegwerfbeutel zur Aufnahme von Fäkalien, insbesondere für die medizinische Analyse, welcher aus einem Blatt aus biologisch abbaubarem Material hergestellt ist, das durch ein WC abgeführt werden kann, wobei das Material so gewählt ist, dass es nicht expandierbar und durchlässig ist für Flüssigkeiten, und welches auf zwei symmetrischen geklappten Seiten (5) auf einer Unterfläche mindestens ein klebendes Element (6) aufweist, das für eine provisorische Befestigung auf einem WC-Becken (1) bestimmt ist, **dadurch gekennzeichnet, dass** der Beutel in der Form eines auffaltbaren und in gefaltetem Zustand flachen Behälters ausgebildet ist und dadurch, dass der Beutel in Form eines Bandes aus einem um sich selbst in zwei Hälften (11, 11') entlang einer rechtwinklig zur Länge des besagten Bandes transversalen Bodenfaltungslinie (12) gefalteten Blattes hergestellt ist, wobei die Längsränder der beiden Hälften des besagten Bandes auf jeder Seite durch ein dreieckiges Element (13, 13') mindestens auf ihrem der Bodenfaltungslinie (12) anliegenden Teil vereinigt sind und wobei jedes der dreieckigen Elemente (13, 13') eine seitliche Faltungslinie(14, 14') aufweist, die entlang der Winkelhalbierenden des an der Bodenfaltungslinie (12) anstossenden Winkels verläuft.

2. Wegwerfbeutel nach Anspruch 1, **dadurch gekennzeichnet, dass** das biologisch abbaubare Material mehrere eingesprengte Schichten aus Vliesfasern , die einen Filz bilden, aufweist.

3. Wegwerfbeutel nach Anspruch 1, **dadurch gekennzeichnet, dass** die Faltung entlang einer seitlichen Faltungslinie (14, 14') nach aussen hin durchgeführt wird.

4. Wegwerfbeutel nach Anspruch 2 oder 3, **dadurch gekennzeichnet, dass** er zwei Längsfaltungslinien (16, 16') aufweist, welche den nicht frei liegenden Seiten des dreieckigen Elements (13, 13') entsprechen , um die dreieckigen Elemente doppelt zu falten, wenn der Beutel sich in gefaltetem Zustand befindet.

5. Wegwerfbeutel nach Anspruch 3, **dadurch gekennzeichnet, dass** jede Hälfte (11, 11') des Bandes eine transversale (15, 15') Umklappfaltungslinie aufweist, welche oberhalb ihres an das dreieckige Element anschliessenden Teil angeordnet ist, um eine geklappte Seite (5) auf der unteren Seite, auf der das klebende Element (6) angeordnet ist, zu erhalten.

6. Wegwerfbeutel nach irgendeinem der vorangegangenen Ansprüche, **dadurch gekennzeichnet, dass** das klebende Element (6) ein doppelseitiges, auf die untere Seite der geklappten Seite (5) geklebtes Klebeband ist.

7. Wegwerfbeutel nach Anspruch 5, **dadurch gekennzeichnet, dass** die obere Seite der umgeklappten Seite (5) einen gefärbten Indikator aufweist.

8. Wegwerfbeutel nach irgendeinem der vorangegangenen Ansprüche 3 bis 8, **dadurch gekennzeichnet, dass** er aus einem bandförmigen Blatt bzw. kontinuierlichen Band (21) aus einem Material besteht, in das man eine symmetrische Kontur in Bezug auf eine der Bodenfaltungslinie (12) entsprechenden Klapp-Transversallinie ausschneidet, wobei die besagte Kontur entlang der besagten Transversallinie (12) gefaltet wird und die seitlichen Ränder der besagten Kontur aneinander befestigt werden.

9. Wegwerfbeutel nach irgendeinem der vorangegangenen Ansprüche 1 bis 9, **dadurch gekennzeichnet, dass** das Material des besagten Blattes aus Cellulosewatte besteht.

## Claims

1. Disposable bag for collecting faecal matter, in particular with a view to medical analyses, made of a sheet of biodegradable material which can be disposed of in toilets, the said material being chosen to be non-extensible and permeable to liquids and which has, on two symmetrical flaps (5), at least one adhesive element (6) on the under surface designed to be temporarily fixed to a toilet bowl (1), **characterized in that** it is configured in the form of a foldable reservoir (4) which is flat in the folded state and **in that** it is made in the form of a band of sheet material folded on itself in two halves (11, 11') along a bottom transverse fold line (12) perpendicular to the length of the said band, the longitudinal edges of the two halves of the said band being joined on each side by a triangular element (13, 13') at least on their part adjacent to the bottom fold line (12), each of the triangular elements (13, 13') having a lateral fold line (14, 14') positioned along the bisector of the angle adjacent to the bottom fold line (12).

2. Disposable bag according to claim 1, **characterized in that** the biodegradable material includes multiple layers of tangled non-woven fibres forming a felt.

3. Disposable bag according to claim 1, **characterized in that** the fold along the lateral fold line (14, 14') is made outwards.

4. Disposable bag according to claim 3 or 4, **characterized in that** it includes two longitudinal fold lines (16, 16') corresponding to the non-free sides of the triangular element (13, 13') so that the triangular elements that are folded in two onto the band can be turned back when the bag is in the folded state.

5. Disposable bag according to claim 3, **characterized in that** each half (11, 11') of the band includes a flap transverse fold line (15, 15') provided above its part connected to the triangular element, so as to determine a flap (5) on the lower face of which the adhesive element (6) is positioned.

6. Disposable bag according to any one of the preceding claims, **characterized in that** the adhesive element (6) is a double-sided adhesive tape glued onto the lower face of the flap (5).

7. Disposable bag according to claim 5, **characterized in that** the upper face of the flap (5) has a coloured indicator (8).

8. Disposable bag according to any one of claims 3 to 8, **characterized in that** it is made from a continuous band or strip of sheet material (21) from which a contour is cut so as to be symmetrical with respect to a transverse line of the strip corresponding to the bottom fold line (12), the said contour being folded along the said transverse line (12) and the lateral edges of the said contour being fixed one on the other.

9. Disposable bag according to any one of claims 1 to 9, **characterized in that** the material of the said sheet is made of cellulose wadding.
